# EUROPEAN PATENT SPECIFICATION

(11) **EP 2 651 458 B1**
(45) Date of publication and mention of the grant of the patent: **13.09.2017**
(21) Application number: 11794802.6
(22) Date of filing: 16.12.2011
(51) Int. Cl.: A61L 9/12, C11D 17/00, C11D 17/04, E03D 9/03, C11D 3/50, E03D 9/00, E03D 9/02

(54) **DEODORISER**
DESODORIERENDES MITTEL
DÉSODORISANT

(30) Priority: 16.12.2010 EP 10290661
(43) Date of publication of application: 23.10.2013
(73) Proprietor: Givaudan SA, 1214 Vernier (CH)
(72) Inventor: BLONDEAU, Philippe, F-75019 Paris (FR); JOUBERT, Caroline, F-92600 Asnières sur seine (FR); BRESSON, BOIL, Alice, F-95220 Herblay (FR)
(74) Representative: McStea, John Anthony
(86) International application number: PCT/EP2011/073085
(87) International publication number: WO 2012/080480

(56) References cited:
- EP-A1- 1 522 319
- WO-A1-2006/005410
- WO-A1-2006/128316
- DE-A1-102008 003 359

## Description

This description relates to devices for disinfecting and deodorising toilets.

One well-known way of deodorising and disinfecting toilet bowls is by hanging a device comprising a soluble block containing an active (typically one or more of a cleaning agent, a disinfectant and a fragrance) in a toilet bowl such that the flush water from the bowl rim washes over it and releases some active. These are generally known as "rim blocks", which term shall hereinafter be used to describe them. Various iterations of the same idea have been used. For example, the block (usually held in a cage, and this refillable) has been replaced by an active-containing gel of natural or synthetic material, with the desired solubility/active leaching characteristics.

The rim blocks have enjoyed wide success, because they are cheap and convenient to use. However, it is also known that their fragrance performance is not as good as desired, especially near the end of the life of the block, when performance falls off sharply. Ideally, the fragrance should stay sufficiently strong until the rim block disappears or needs to be changed. No block currently on the market does this. There have been attempts to overcome the problem, such as combining the rim block with a polyolefin membrane-based air freshener. However, this type of membrane is known to have major selectivity issues leading to fragrance restrictions when fragrancing this sort of system. Another attempted solution was to combine the rim block with a fragranced gel, but here again there are technical drawbacks as these gels are not water-soluble (no end of life signal) and their olfactory performance falls off significantly before mid-life. WO 2006/128316 discloses a volatile dispensing apparatus comprising an opening being closed by a permeable membrane, which is not in contact with the liquid in the reservoir when the apparatus is in operation, but which is capable of absorbing a quantity of liquid that will evaporate when the membrane is brought into direct contact with the liquid. It has now been found that it is possible to make a block that can perform in this desirable fashion. There is therefore provided a device that is adapted to add active to the water of a flushed toilet and fragrance to the air in its vicinity, comprising, in a single unit
(a) water-soluble rim block comprising active;
(b) fragrance dispenser, and
(c) means for holding (a) and (b) in the path of flush water from a toilet bowl rim; characterised in that the fragrance dispenser comprises a container with an opening covered by a membrane having a thickness of from 0.05-1.0mm and consisting essentially of a homogeneous mixture of 8 to 98 vol. % polyolefin having a molecular weight (weight-average) of at least 300,000, a standard load melt index of less than 0.1 and a reduced viscosity of not less than 4.0, 1 to 30 vol. % filler and 1 to 40 vol. % plasticizer, the fragrance dispenser being orientated such that the fragrance is permanently in contact with the membrane.

There is additionally provided a method for both deodorising water in a toilet bowl and providing fragrance in the bowl, comprising the location in the path of flush water of a device comprising both a water-soluble rim block and a fragrance dispenser, the fragrance dispenser comprising a membrane having a thickness of from 0.05-1.0mm and consisting essentially of a homogeneous mixture of 8 to 98 vol. % polyolefin having a molecular weight (weight-average) of at least 300,000, a standard load melt index of less than 0.1 and a reduced viscosity of not less than 4.0, 1 to 30 vol. % filler and 1 to 40 vol. % plasticizer, the fragrance dispenser being orientated such that the fragrance is permanently in contact with the membrane.

The rim block is similar to known rim blocks, consisting typically of a cage of any suitable material and containing a block, either of a solid material, or a gel, also in solid form or in a membrane enclosure that allows contact with water. Typical gels are made of xanthan gum Carbopol, or cellulose derivatives. The block may contain any desirable active in a typical art-recognised concentration. Typical actives (more than one may be present) include cleaning agents, detergents and surfactants, fragrances, disinfectants, bactericides and malodour counteractants.

The fragrance dispenser may be any suitable container of any suitable material that does not affect or is not affected by fragrance. It may be, for example, plastics, metal, ceramic or composite material. Its dimensions are not narrowly critical, provided they permit both an adequate charge of fragrance and the ability to fit in a toilet bowl. It may be fitted to the block itself or to the cage holding the block. The factors determining the actual arrangement include whether the device is a once-only disposable device or a refillable device, but the skilled person can easily provide a suitable arrangement for any particular configuration.

The fragrance container has an opening that is covered by a membrane with which the fragrance is in permanent contact. This can be arranged in any convenient way. For example, the dispenser may include a porous wick, which contacts both liquid and membrane, and through which fragrance is conveyed to the membrane and thus to the atmosphere. However, in a particular embodiment, the liquid contacts the membrane directly. Again, this can be by any suitable arrangement, a typical one being a container having the form of a shallow cylinder, open at one end, the open end being closed with the membrane and the container being arranged such that the membrane is vertical. With the particular membranes involved here, it is possible to have the container upside-down, so that the membrane forms the floor of the container. The membrane may be attached to the container in any convenient manner, for example, by being adhered to it, or by being part of a screw-on lid.

The membrane consists essentially of a homogeneous mixture of 8 to 98 vol. % polyolefin having a molecular weight (weight-average) of at least 300,000, a standard load melt index of less than 0.1 and a reduced viscosity of not less than 4.0, from 1 to 30 vol. % filler and 1 to 40 vol. % plasticizer. Suitable fillers and plasticizers are known to the art. In this context, reference is made to U.S Pat.No.3,351,495. A particular filler is finely-divided silica (silicic acid).The average particle size (diameter) of the filler is the range from 0.01 to about 20 .mu.m (milliatomic mass unit times metre) the surface area of the filler being in the range from 30 to 950m²/g, and particularly at least 100m²/g. Other fillers that may be used include various mineral fillers, such as clays, zeolites and carbonates, and charcoal.

The membrane is particularly of 0.25-0.7mm thickness, more particularly about 0.6mm

Such membranes are known to the art, being used, for example, as battery separators. Commercial examples are sold by, for example, Daramic, Inc.

Membranes as hereinabove defined have a number of very useful characteristics. One is that they do not allow the passage of water through them. They are thus unaffected by water running over them when a toilet is operated. Another is that they allow the passage of alcohol-type fragrance materials. Many of the most common fragrance materials have alcohol functionality, for example, linalool and dihydromyrcenol, and other membranes do not readily allow these through. The result is that the desired olfactory effect of a carefully-formulated fragrance is not achieved, and perfumes for use with membranes previously use by the art (typically LLDPE/EVOH/ LLDPE laminates of about 50 to 100 micron thickness) had to be specifically formulated to allow for this. Moreover, the technical restrictions linked to this type of membrane do not allow the perfumers to create in all olfactive directions, for example, real performing lavender fragrances are impossible to create. The membranes hereinabove described will allow the passage of alcohol fragrance materials, and so there is no need specifically to formulate a fragrance for this use. An unusually wide spectrum of fragrances may therefore be used.

In addition, the membranes have the feature of colour change when the fragrance is exhausted, thus giving a convenient end-of-life indication.

In addition, the membranes hereinabove described are unaffected by water. Thus, the flow of flush water in a toilet bowl across the membrane has no effect on its performance. They do not absorb the water, so there is no risk of contamination of the fragrance in the container. The orientation of the membrane with respect to the flow of flush water is therefore not important and the membrane may be positioned in any desired orientation, depending on other practical or aesthetic considerations.

The means for holding the device in the path of flush water may be any convenient device. It is typically a hook, which hooks over the rim of a toilet bowl and which, at its other end, is attached to the device, the dimensions being such as to allow proper positioning. The hook may be integral with the device (for example, moulded with a plastics cage as one piece), or it may be a separate piece, able to be attached to the device when the latter is ready to be put into service. The appropriate selection of materials, dimensions and geometry are all within the ordinary skill of the art.

The devices hereinabove defined offer substantial advantages over existing products. They permit a prolonged effectiveness, which lasts throughout the service life of the device, plus the convenience of an end-of-life indication for the fragrance dispenser.

The device is now further defined with respect to the accompanying drawings, which depicts particular embodiments, and which are not intended to be in any way limiting.
Figure 1 is a schematic transverse cross-section of a particular embodiment.
Figure 2 is a schematic part transverse cross-section of a further particular embodiment.

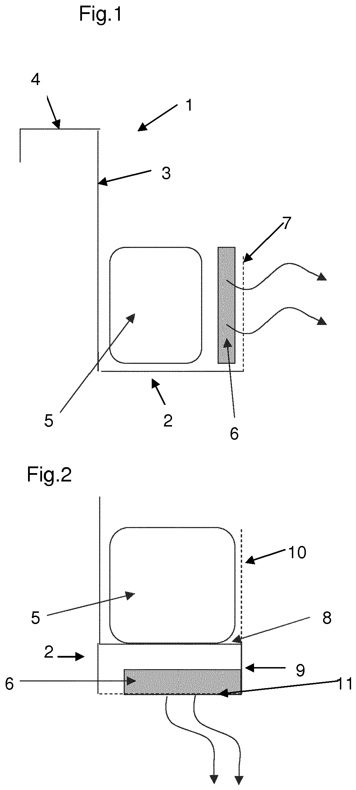

In Figure 1, a device generally indicated as 1 comprises a cage 2 in the form of a tray from which extends upwardly a support member 3, which ends in a hook member 4, adapted to hook around a rim of a toilet bowl, the support member 3 being of such a length that the cage 2 lies in the path of flush water from the rim. That side of the cage 7 opposite to the support member is perforated, so that flush water can escape through it. The elements 2, 3, 4 and 7 are of a plastics material and are moulded in a single piece.

Within the tray are seated a toilet block 5 and a fragrance dispenser 6. The toilet block is a block of the type well known to the art, consisting of anionic and non-ionic surfactants, principally alkyl benzene sulphonates and ethoxylated fatty alcohols, fillers/builders, such as sodium sulphate and chelating agents to counter the effects of hardness in water. The fragrance content is 5% by weight of the block.

The fragrance dispenser 6 has the form of a shallow cylindrical dish of plastics material and contains 10ml perfume. The membrane, which is bonded to the open face of the dish is Membrane DS2 drying sweat system (ex Daramic, Inc) of 1mm thickness.

The dispenser 6 is filled with perfume and is seated in the cage 2 beside the block, such that the membrane is substantially vertical, and that the perfume within is in permanent contact therewith. The dispenser is dimensioned, so that it fits easily within the cage, but is located sufficiently firmly that it will not be dislodged by the flow of flush water.

In operation, the flush water flows into the tray and out again through the perforated side 7, taking with it cleaning materials from the block 5. When the water flow has subsided, the fragrance dispenser dispenses fragrance through the membrane and also through the perforated side 7, as depicted by the wavy lines in Figure 1.

The membrane described has the characteristic of changing colour when the fragrance is exhausted. This provides am end-of-life signal, allowing for the replacement of the fragrance dispenser.

In Figure 2, the members 3 and 4 are the same as in Figure 1, but the tray 2 is divided into an upper portion 8 and a lower portion 9, the lower portion being located vertically below the upper portion when the device is positioned in a toilet bowl. A toilet block 5 resides in the upper portion and a fragrance dispenser 6 in the lower portion 9. A side 10 of the upper portion opposite the support member is perforated, the perforations extending to the boundary of the upper and lower portions. The lower portion also has a perforated face 11, this being located at the base of the lower portion 9, such that it faces downwards into the toilet bowl. In this case, the fragrance dispenser 6 is places such that the membrane lies against the perforated face 11.

The operation of the Figure 2 device is similar to that of Figure 1, except in this case fragrance emission is continuous, even when a flush is in progress.

## Claims

1. A device that is adapted to add active to the water of a flushed toilet and fragrance to the air in its vicinity, comprising, in a single unit
(a) water-soluble rim block comprising active;
(b) fragrance container, and
(c) means for holding (a) and (b) in the path of flush water from a toilet bowl rim;
**characterised in that** the fragrance dispenser comprises a container with an opening covered by a membrane having a thickness of from 0.05-1.0mm and consisting essentially of a homogeneous mixture of 8 to 98 vol. % polyolefin having a molecular weight (weight-average) of at least 300,000, a standard load melt index of less than 0.1 and a reduced viscosity of not less than 4.0, 1 to 30 vol. % filler and 1 to 40 vol. % plasticizer, the fragrance dispenser being orientated such that the fragrance is permanently in contact with the membrane, and **in that** the fragrance contains fragrance materials with alcohol functionality.

2. A device according to claim 1, in which the average particle size (diameter) of the filler is the range from 0.01 to about 20 .mu.m, the surface area of the filler being in the range from 30 to 950m²/g, more preferably at least 100m²/g.

3. A device according to claim 1, in which the filler is finely-divided silica (silicic acid).

4. A device according to claim 1, in which the membrane is from 0.25-0,7mm thick.

5. A device according to claim 1, in which the fragrance comprises alcohol components.

6. A method for both deodorising water in a toilet bowl and providing fragrance in the bowl, comprising the location in the path of flush water of a device comprising both a water-soluble rim block and a fragrance dispenser, the fragrance dispenser comprising a membrane having a thickness of from 0.05-1.0mm and consisting essentially of a homogeneous mixture of 8 to 98 vol. % polyolefin having a molecular weight (weight-average) of at least 300,000, a standard load melt index of less than 0.1 and a reduced viscosity of not less than 4.0, 1 to 30 vol. % filler and 1 to 40 vol. % plasticizer, the fragrance dispenser being orientated such that the fragrance is permanently in contact with the membrane, and in that the fragrance contains fragrance materials with alcohol functionality.

## Patentansprüche

1. Vorrichtung, die ausgelegt ist, dem Wasser einer gespülten Toilette einen Wirkstoff und der Luft in ihrer Umgebung einen Duftstoff hinzuzufügen, umfassend, in einer einzelnen Einheit
(a) einen wasserlöslichen Beckenstein, der einen Wirkstoff umfasst;
(b) einen Duftstoffbehälter; und
(c) ein Mittel zum Halten von (a) und (b) im Weg des Spülwassers von einem Toilettenschüsselrand;
**dadurch gekennzeichnet, dass** der Duftspender einen Behälter mit einer Öffnung umfasst, die von einer Membran mit einer Dicke von 0,05-1,0 mm abgedeckt ist, die im Wesentlichen aus einem homogenen Gemisch von 8 bis 98 Vol.-% Polyolefin mit einem Molekulargewicht (Gewichtsmittel) von mindestens 300.000, einem Standard-Lastschmelzindex von weniger als 0,1 und einer reduzierten Viskosität von mindestens 4,0, 1 bis 30 Vol.-% Füllstoff und 1 bis 40 Vol.-% Weichmacher besteht, wobei der Duftspender derart orientiert ist, dass der Duftstoff permanent mit der Membran in Kontakt ist, und dass der Duftstoff Duftstoffe mit Alkoholfunktionalität enthält.

2. Vorrichtung nach Anspruch 1, in der die mittlere Teilchengröße (Durchmesser) des Füllstoffs im Bereich von 0,01 bis ungefähr 20 .mu.m liegt, wobei die Oberfläche des Füllstoffs im Bereich von 30 bis 950 m²/g liegt und bevorzugter mindestens 100 m²/g beträgt.

3. Vorrichtung nach Anspruch 1, in der der Füllstoff feinverteiltes Siliciumdioxid (Kieselsäure) ist.

4. Vorrichtung nach Anspruch 1, in der die Membran 0,25-0,7 mm dick ist.

5. Vorrichtung nach Anspruch 1, in der der Duftstoff Alkoholbestandteile umfasst.

6. Verfahren zum Desodorisieren von Wasser in einer Toilettenschüssel und zum Bereitstellen eines Duftstoffs in der Schüssel, umfassend die Anordnung, im Weg des Spülwassers, einer Vorrichtung, die sowohl einen wasserlöslichen Beckenstein als auch einen Duftspender umfasst, wobei der Duftspender eine Membran mit einer Dicke von 0,05-1,0 mm umfasst, die im Wesentlichen aus einem homogenen Gemisch von 8 bis 98 Vol.-% Polyolefin mit einem Molekulargewicht (Gewichtsmittel) von mindestens 300.000, einem Standard-Lastschmelzindex von weniger als 0,1 und einer reduzierten Viskosität von mindestens 4,0, 1 bis 30 Vol.-% Füllstoff und 1 bis 40 Vol.-% Weichmacher besteht, wobei der Duftspender derart orientiert ist, dass der Duftstoff permanent mit der Membran in Kontakt ist, und dass der Duftstoff Duftstoffe mit Alkoholfunktionalität enthält.

## Revendications

1. Dispositif conçu pour ajouter une substance active à l'eau de toilettes quand la chasse est tirée et un parfum dans l'air dans sa proximité, comprenant, dans une seule unité :
(a) un bloc hydrosoluble à placer sur le rebord, comprenant la substance active ;
(b) un contenant de parfum, et
(c) un moyen pour maintenir (a) et (b) dans le chemin d'écoulement de l'eau de chasse à partir d'un rebord de cuvette des toilettes ; **caractérisé en ce que** le distributeur de parfum comprend un contenant pourvu d'une ouverture recouverte d'une membrane ayant une épaisseur de 0,05 à 1,0 mm et consistant essentiellement en un mélange homogène de 8 à 98 % en volume d'une polyoléfine ayant une masse moléculaire (moyenne en poids) d'au moins 300 000, un indice de fusion sous charge mesuré par une méthode normalisée qui est inférieur à 0,1 et une viscosité réduite qui n'est pas inférieure à 4,0, de 1 à 30 % en volume d'une charge et de 1 à 40 % en volume d'un plastifiant, le distributeur de parfum étant orienté de telle sorte que le parfum soit constamment en contact avec la membrane, et **en ce que** le parfum contient des matériaux de parfum ayant une fonctionnalité alcool.

2. Dispositif selon la revendication 1, dans lequel la taille de particule moyenne (diamètre) de la charge est de 0,01 à environ 20 .mu.m, l'aire de surface de la charge étant de 30 à 950 m²/g, plus préférablement d'au moins 100 m²/g.

3. Dispositif selon la revendication 1, dans lequel la charge se compose de silice finement broyée (acide silicique).

4. Dispositif selon la revendication 1, dans lequel la membrane a une épaisseur de 0,25 à 0,7 mm.

5. Dispositif selon la revendication 1, dans lequel le parfum comprend des constituants alcools.

6. Procédé permettant à la fois de désodoriser l'eau dans une cuvette de toilettes et de libérer un parfum dans la cuvette, comprenant le positionnement dans le chemin d'écoulement de l'eau de chasse d'un dispositif comprenant un bloc hydrosoluble à placer sur le rebord ainsi qu'un distributeur de parfum, le distributeur de parfum comprenant une membrane ayant une épaisseur de 0,05 à 1,0 mm et consistant essentiellement en un mélange homogène de 8 à 98 % en volume d'une polyoléfine ayant une masse moléculaire (moyenne en poids) d'au moins 300 000, un indice de fusion sous charge mesuré par une méthode normalisée qui est inférieur à 0,1 et une viscosité réduite qui n'est pas inférieure à 4,0, de 1 à 30 % en volume d'une charge et de 1 à 40 % en volume d'un plastifiant, le distributeur de parfum étant orienté de telle sorte que le parfum soit constamment en contact avec la membrane, et en ce que le parfum contient des matériaux de parfum ayant une fonctionnalité alcool.
